Europäisches Patentamt

European Patent Office

Office européen des brevets

(11) Publication number: **0 150 250**
**B1**

(12) **EUROPEAN PATENT SPECIFICATION**

(45) Date of publication of patent specification: **10.08.88**

(51) Int. Cl.[4]: **A 61 K 7/50**

(21) Application number: **84101078.8**

(22) Date of filing: **02.02.84**

(54) Gas-emitting bath additive composition.

(43) Date of publication of application:
**07.08.85 Bulletin 85/32**

(45) Publication of the grant of the patent:
**10.08.88 Bulletin 88/32**

(84) Designated Contracting States:
**AT CH DE FR GB IT LI SE**

(56) References cited:
**FR-A- 722 490**
**US-A-3 584 099**

**PATENTS ABSTRACTS OF JAPAN, vol. 4, no.
34 (C-3)516r, 22nd March 1980; & JP - A - 55
7246 (KANEBO K.K.) 19-01-1980**

(73) Proprietor: **Kao Corporation
14-10, Nihonbashi Kayabacho 1-chome
Chuo-Ku Tokyo 103 (JP)**

(72) Inventor: **Eguchi, Yasuteru
513-6, Mine-machi
Utsunomiya-shi Tochigi-ken (JP)**
Inventor: **Yorozu, Hidenori
1729, Ooaza-Mashiko Mashiko-machi
Haga-gun Tochigi-ken (JP)**
Inventor: **Iijima, Eiji
4880-71, Miyuki-honmachi
Utsunomiya-shi Tochigi-ken (JP)**

(74) Representative: **Wächtershäuser, Günter, Dr.
Tal 29
D-8000 München 2 (DE)**

Courier Press, Leamington Spa, England.

## Description

### i) Field of the Invention:

This invention relates to gas-emitting bath additive compositions which are stably preserved over a long period of time.

### ii) Description of the Prior Art:

Bath additives are usually made of mixtures of inorganic salts or materials such as Glauber's salt, borax, sulfur, sodium chloride, carbonates and the like, admixed with perfumes, colorants, plant extracts, organic acids and the like. These additives serve to impart agreeable fragrance and color to a hot bath and also to give a proper impetus to skin, thus promoting the circulation of blood and leading to recruitment and promotion of metabolism. Among these bath additives, there are known gas-emitting bath additives comprising combinations of carbonates and organic acids. This type of gas-emitting bath additive allows carbon dioxide gas to be generated or emitted in or throughout a hot bath, producing the effect of permitting one to relax and feel refreshed while enjoying the bath.

However, even though anhydrous carbonates and anhydrous organic acids are used in these gas-emitting bath additives, they are so unstable as to react even in the presence of a very small amount of moisture or water, causing carbon dioxide to be generated at the time when it is not desired. Accordingly, even when such gas-emitting bath additives are tightly packed in a container and kept over a long term, the container is expanded by the action of carbon dioxide gas generated. This results in poor appearance of the container and a lowering of commercial value, with the attendant disadvantage that the satisfactory gas-emitting effect cannot be achieved because of a lowering of gas-emitting ability.

In order to overcome the disadvantages, attempts have been made, without practical success, to use moisture absorbents such as anhydrous sodium sulfate, starch and the like.

US—A—3 584 099 discloses a composition comprising a mixture of a carbonate, an organic acid and magnesium oxide as a stabilizer for use as an effervescent mouthwash.

### Summary of the Invention

We have now made intensive studies on gas-emitting bath additives and, as a result, found that the use of magnesium oxide and/or sodium aluminate leads to gas-emitting bath additives which are stable with respect to storage.

According to the present invention a composition comprising a mixture of a carbonate, an acid selected from phosphoric acid, organic acid and the salt thereof, and a stabilizer selected from magnesium oxide and sodium aluminate is used as a gas-emitting bath additive.

### Detailed Description of the Invention and Preferred Embodiments

The carbonates used in the gas-emitting bath additive of the present invention include, for example, dried sodium hydrogencarbonate, sodium carbonate, sodium sesquicarbonate, potassium carbonate, potassium hydrogencarbonate and ammonium carbonate. Of these, sodium hydrogencarbonate and sodium carbonate are preferred. The content of the carbonate is in the range of 5—80 wt%, preferably 10—50 wt%, of the total weight of the composition.

The acid includes phosphoric acid, organic acid and the salt thereof.

The organic acids are, for example, dried citric acid, tartaric acid, malic acid, malonic acid, pyridone-carboxylic acid, succinic acid, fumaric acid and the like.

The acids' salts are, for example, sodium succinate, sodium fumarate and sodium phosphate.

The acids are suitably used depending on the desired effect of the bath additive. The amount of the organic acid is in the range of 10—300 wt%, preferably 30—150 wt%, of the weight of the carbonate present in the bath composition.

When a bath additive comprising a carbonate and an organic acid is added to a hot bath, carbon dioxide gas generates by reaction. The effect of the generated carbon dioxide gas depends on the pH of the hot bath. When the hot bath is acidic, carbon dioxide gas exists as $CO_2$ molecules, showing the action of promoting the blood circulation. On the contrary, when a hot bath has a pH on the alkaline side, such an effect as mentioned above is not developed because carbon dioxide gas is present as $CO_3^{2-}$ or $HCO_3^-$. From this, it is favorable to control the mixing ratio of the carbonate and organic acid in such a way that the hot bath is weakly acidic when the gas-emitting bath additive is charged thereinto, e.g. an aqueous solution of 0.01 wt% of the bath additive has a pH of 5—7. The weakly acidic hot bath is close to the pH of the skin and gives a favorable influence on the skin. In order to attain the weak acidity, the content of organic acids are preferably as follows: it is in the range of 20—150 wt% of carbonate (calculated as sodium hydrogencarbonate) for succinic acid or fumaric acid, in the range of 40—300 wt% of carbonate for sodium succinate, and in the range of 30—250 wt% of carbonate for sodium fumarate.

On the other hand, in case where the bath additive of the present invention is expected to emit or generate carbon dioxide gas without expecting any pharmacological effect, the two ingredients may be mixed to have a neutral to weakly alkaline pH range. In the case, it is preferable that a mixing ratio of an organic acid to a carbonate is smaller than in the abovementioned case.

In the practice of the invention, magnesium oxide and/or sodium aluminate which is used as a stabilizer is used in an amount ranging 0.5—15 wt%, preferably 0.5—10 wt%, based on the total weight of the composition. Amounts less than 0.1 wt% are unsatisfactory in the stabilizing effect, whereas amounts exceeding 15 wt% are unfavorable because several disadvantages arise, e.g. the hot bath becomes very cloudy or floating matter is produced or the soap hardly lathers.

The gas-emitting bath additive composition of the invention may further comprise, apart from the above-indicated ingredients, sulfates such as sodium sulfate, magnesium sulfate and zinc sulfate, chlorides such as sodium chloride, by which there are obtained effects of hot springs such as Glauber's salt hot spring, sulfate hot spring, salt hot spring. The amounts of these compounds are, in combination, in the range of 30—500 wt%, preferably 50—200 wt%, of the total weight of a carbonate, an organic acid and a stabilizer used.

The gas-emitting bath additive of the invention may further comprise perfumes, colorants and, if desired, vitamins, effective components from hot spring, proteinases, seaweed extracts, sodium aluginate, lanolin, silicones, amphoteric active agents, and crude drugs or extracts thereof.

The gas-emitting bath additive may be formed as powder, granules, crystals and tablets. Among them, tablets are the most preferable from the viewpoint of gas absorption. For the preparation, any known excipients, binders disintegrators, lubricants and the like may be added as usual. The prepared or shaped gas-emitting bath additive may be packed in a packaging material substantially impermeable to water, e.g. in an aluminium laminate film, for single usage. Alternatively, such additive may be placed in a sealed container for commercial purposes.

As will be appreciated from the foregoing, the gas-emitting bath additive of the invention is stable over a long period of time and readily dissolves in a hot bath, thus being easy to handle. In addition, it generates or emits gas to an adequate degree, which serves to refresh or relax one in the bath. The bath additive which is controlled to be weakly acidic has the effect of promoting the circulation of blood by its vasodilating action.

The present invention is illustrated by way of example, in which parts are by weight.

### Example 1

Fifty parts by weight of sodium hydrogencarbonate, 50 parts by weight of citric acid, 1 part of magnesium oxide, 1 part of a perfume, and a suitable amount of a colorant were placed in a powder mixer and mixed sufficiently. The mixture was shaped in a tableting machine to obtain tablets having a diameter of 3 cm and a thickness of 1 cm. The tablets were tightly packed in an aluminium laminate film and kept at a temperature of 40°C in a relative humidity of 75% for 6 months. The packed tablets suffered no change. Upon adding of the tablets to a hot bath, it could generate gas to a suitable degree, giving a massotherapeutical effect on the skin and permitting one to be refreshed.

### Example 2

Seventy parts of sodium hydrogencarbonate, 30 parts of tartaric acid, 1 part of magnesium oxide, 1 part of a perfume and a suitable amount of a colorant were placed in a powder mixer and sufficiently mixed to give a powdery gas-emitting bath additive. This powder was packed in the same way as in Example 1 and kept at a temperature of 40°C in a relatively humidity of 75% for 6 months. The packed powder suffered no change. When the powder was added to a hot bath, bubbles were generated intensely giving a massotherapeutical effect on the skin and permitting one to be refreshed.

### Example 3

Seventy parts of sodium carbonate, 30 parts of citric acid, 5 part of sodium aluminate, 2 part of a perfume, and a suitable amount of colorant were used and treated in the same manner as in Example 1, thereby obtaining a powdery gas-emitting bath additive. This additive had the same storage stability and massotherapeutical effect as in the case of Example 1.

### Example 4

Sixty parts of sodium sesquicarbonate, 40 parts of citric acid, 0.5 part of sodium aluminate, 1 part of a perfume, and a suitable amount of a colorant were used and treated in the same manner as in Example 2, thereby obtaining a powdery gas-emitting bath additive. This additive had the same storage stability and effect as in the case of Example 1.

### Example 5

Fifty parts of sodium hydrogencarbonate, 55 parts of citric acid, 2 parts of magnesium oxide, 1 part of a perfume, and a suitable amount of a colorant were used and treated in the same manner as in Example 1, thereby obtaining tablets of gas-emitting bath additive. The additive had the same storage stability as in the case of Example 1. Upon adding of the tablets to a hot bath, they were found to emit gas bubbles so that the massotherapeutical effect was given to the skin while showing the blood-circulation effect. The pH of a 0.01 wt% aqueous solution of the bath additive was 5.4.

### Example 6

Thirty parts of sodium hydrogencarbonate, 40 parts of succinic acid, 25 parts of sodium sulfate, 2 parts of sodium aluminate, 1 part of a perfume, and a suitable amount of a colorant were used and treated in the same manner as in Example 1, thereby obtaining a gas-emitting bath additive in the form of tablets. This additive had the same storage stability as that of Example 1. When the additive was added to a hot bath, it turned into a kind of carbonate or salt cake spring. A 0.01 wt% aqueous solution of the gas-emitting bath additive had a pH of 6.5.

### Example 7

Forty parts of sodium sulfate, 30 parts of sodium hydrogencarbonate, 30 parts of sodium phosphate, 2 parts of magnesium oxide, 1 part of a perfume, and a suitable amount of a colorant were used and treated in the same manner as in Example 1, thereby obtaining tablets of gas-emitting bath additive. This additive had the same storage stability and effect as in the case of Example 1.

### Example 8

Seventy parts of sodium hydrogencarbonate, 30 parts of fumaric acid, 2 parts of magnesium oxide, 1 part of sodium aluminate, 1 part of a perfume, a suitable amount of a colorant, and 0.5 part of sodium CMC were used and treated in the same manner as in Example 1, thereby obtaining tablets of gas-emitting bath additive. This additive had the same storage stability and effect as in the case of Example 1.

### Comparative Example

Examples 1—7 were repeated without use of magnesium oxide or sodium aluminate, thereby obtaining gas-emitting bath additives. These additives were kept in the same manner as in the foregoing examples, with the result that carbon dioxide gas generated in 3 days to 2 weeks and the packaging containers were each considerably expanded.

### Claims

1. The use of a composition comprising a mixture of a carbonate, an acid selected from phosphoric acid, organic acid and the salt thereof, and a stabilizer selected from magnesium oxide, sodium aluminate and a mixture thereof as a gas-emitting bath additive.

2. The use of the composition as a gas-emitting bath additive according to claim 1, wherein the amount of said stabilizer is in the range of 0.5 to 15 wt% based on the total composition.

### Patentansprüche

1. Verwendung einer Zusammensetzung, umfassend ein Gemisch eines Carbonats, einer Säure, ausgewählt aus Phosphorsäure, organischer Säure und Salzen derselben, und einen Stabilisator, ausgewählt aus Magnesiumoxid, Natriumaluminat und einer Mischung derselben, als gasentwickelnder Badezusatz.

2. Verwendung der Zusammensetzung als gasentwickelnder Badezusatz gemäß Anspruch 1, wobei die Menge des Stabilisators im Bereich von 0,5 bis 15 Gew.-%, bezogen auf die Gesamtzusammensetzung, liegt.

### Revendications

1. Utilisation d'une composition comprenant un mélange d'un carbonate, d'un acide choisi parmi les acides phosphoriques, les acides organiques et leurs sels, et d'un stabilisant choisi parmi l'oxyde de magnésium, l'aluminate de sodium et leurs mélanges, en tant que produit effervescent d'addition aux bains.

2. Utilisation de la composition comme produit effervescent d'addition aux bains selon la revendication 1, dans laquelle la quantité dudit stabilisant se situe dans la plage de 0,5 à 15% en poids, sur la base de la composition totale.